# EUROPEAN PATENT SPECIFICATION

(11) **EP 2 907 541 B1**
(45) Date of publication and mention of the grant of the patent: **07.12.2016**
(21) Application number: 15151619.2
(22) Date of filing: 19.01.2015
(51) Int. Cl.: A61N 1/37, A61N 1/05, A61B 5/0452

(54) **Device and method for lead failure detection**
Vorrichtung und Verfahren zur Leitungsfehlererkennung
Dispositif et procédé de détection de panne de connexion

(30) Priority: 13.02.2014 US 201461939281 P
(43) Date of publication of application: 19.08.2015
(73) Proprietor: BIOTRONIK SE & Co. KG, 12359 Berlin (DE)
(72) Inventor: Stäuber, Philipp, 10827 Berlin (DE); Neumann, Andreas, 12437 Berlin (DE); Busch, Ulrich, 10318 Berlin (DE); Wohlgemuth, Peter, 09123 Chemnitz (DE); Bauditz, Sabrina, 10247 Berlin (DE)
(74) Representative: Galander, Marcus

(56) References cited:
- EP-A1- 2 476 457
- WO-A1-2011/072286
- WO-A2-2006/076498

## Description

The present invention relates to implantable medical devices, and, more particularly, implantable medical devices utilizing leads.

Implantable medical devices (IMD) for delivering a therapy or monitoring a physiologic condition of a human or animal body employ one or more elongated electrical leads in contact with the body tissue. Such IMD can monitor or deliver therapy to the heart, muscle, nerve, brain, and stomach or other organs. IMD such as pacemakers and implantable cardioverter defibrillators (ICD) for example are treating cardiac arrhythmias by delivering electrical impulses to the heart. Such devices comprise sensing units that sense electrical cardiac activities through cardiac leads having one or more electrodes. When an abnormal rhythm is detected, an appropriate electrical therapy is delivered by a therapy unit connected to cardiac leads.

Leads associated with such IMD typically include a lead body extending between a proximal lead end and a distal lead end. The lead body incorporates one or more exposed electrode or sensor elements located at or near the distal lead end. One or more elongated electrical conductors extend through the lead body from a connector assembly provided at a proximal lead end for connection with an associated IMD and an electrode located at the distal lead end or along a section of the lead body. Each electrical conductor is typically electrically isolated from any other electrical conductors and is encased within an outer sheath that electrically insulates the lead conductors from body tissue and fluids. Implantable medical leads typically extend from an implantation site of the IMD through an internal body pathway to a desired tissue site. The leads are generally preferred having small diameter, highly flexible, reliable lead bodies that withstand degradation by body fluids and body movements that apply stress and strain to the lead body and the connections made to electrodes. As lead bodies are made smaller and smaller and/or the number of lead conductors is increased, the integrity of lead conductors is increasingly important. Implantable medical leads that extend to or in the heart (cardiac leads) are continuously flexed by the beating of the heart. Other stresses are applied to the lead body during an implantation or lead repositioning procedure, and by movements of the patient. For that reasons the lead can be slightly damaged, and the slight damage can progress until a lead conductor fractures and/or the insulation is breached causing an interruption of the electric conduction path or a short between conductors that are normally isolated. Also the connection of the lead to the IMD at the connector assembly may be interrupted or shortened. These effects can progress from an intermittent manifestation to a more continuous effect and can be referred to as "lead failures".

Lead failures adversely impact the normal operation of the IMD. Any interruption or short impedes sensing of electrical signals from the tissue and the stimulation of the tissue with electrical pulses. In the case of cardiac leads an interruption or a short may be misinterpreted by the IMD as intrinsic activity of the heart. This is known as oversensing or undersensing and can result in an incorrect interpretation of the cardiac data potentially resulting in inappropriate withholding or delivery of electrical therapy.

Several methods have been developed to monitor lead integrity and detect lead failures. The most common method is the monitoring of lead impedance as significant changes of +lead impedance are an indicator of lead failures. Impedance monitoring consumes energy and may interfere the sensing and is therefore not suited for continuous monitoring. In the case of discontinuous impedance monitoring intermittent lead failures may remain undetected.

Another method analyzes the intra cardiac electrogram (IEGM) waveform by comparing them with reference waveforms that represent physiological signals. This method requires the generation of reference waveforms. Due to the large variance of physiological waveforms this method is susceptible for miss-detections.

A method according to the preamble of claim 1 is known from EP2476457.

Thus there is a need for an improved method and device for detection of lead failures.

Present invention provides a method and a device for detection of lead failures based on an evaluation of detected interferenced electrical activity of the human or animal body.

The invention is based on the finding that lead failures, in particular intermittent lead failures, cause an step response of the first filter in the processing chain of electrical signals detected by an electrode of the lead. This step response is different to physiological signals and can be easily detected in the signal after the first filter. However, any subsequent filtering as usual in common state of the art designs before signal evaluation changes the signal morphology such, that the step response vanishes and the lead failure signal is more similar to physiological signals.

A method to detect a lead failure condition for a lead having at least one electrode in contact with body tissue comprises the steps of:
- sensing a electric signal from the body tissue using said electrode,
- filtering the sensed electric signal using a first filter to a first filtered signal,
- detecting signal characteristics of the first filtered signal and
- indicating a lead failure condition if said detected signal characteristics correspond to the step response of said first filter.

Preferably the first filter is a high-pass filter. The step of detecting signal characteristics may comprise the detection of an abrupt change of the signal amplitude and the detection of an exponential return of the signal amplitude to a previous mean signal amplitude. The abrupt change or step of the signal amplitude is caused by a short or interrupt characteristic for a lead failure. The exponential return of the signal amplitude to a previous mean signal amplitude is the characteristic step response of a high-pass filter. The detection of an abrupt change of the signal amplitude may be performed by analyzing the slew rate of the signal amplitude. In a preferred embodiment, the method comprises the detection of a minimum slew rate of the signal amplitude. Alternatively the signal amplitude is compared to one or more pre-determined thresholds or amplitude ranges at one or more pre-determined times or during pre-determined time windows. An exponential return of the signal amplitude to a previous mean signal amplitude may be detected by comparing the signal amplitude to the known step response of the first filter for the respective amplitude step. As the return of the signal amplitude of a body signal to a previous mean signal amplitude is significantly shorter than the return in case of a step response, a simplified method to detect the exponential return comprises the step of determination whether the time between the abrupt change of the signal amplitude and the return to a previous mean signal amplitude exceeds a predetermined time.

The method can comprise the additional steps of:
- filtering the first filtered signal using a second filter to a second filtered signal,
- detecting body signals in the second filtered signal and
- marking detected body signals as invalid if a lead failure condition is indicated.

Preferably the method is applied to electric signals that are cardiac signals.

The device is configured to detect a lead failure condition for a lead having at least one electrode in contact with body tissue wherein the lead is connected to the device. The device comprises at least a first filter to filter a electric signal sensed by the at least one electrode to a first filtered signal and a lead failure detection unit that is configured to detect signal characteristics of the first filtered signal. A lead failure condition is indicated by the lead failure detection unit if the detected signal characteristics correspond to the step response of the first filter.

Preferably the first filter is a high-pass filter. The signal characteristics comprise an abrupt change of the signal amplitude and an exponential return of the signal amplitude to a previous mean signal amplitude. An abrupt change of the signal amplitude is detected by by analyzing the slew rate of the signal amplitude. In a preferred embodiment, a minimum slew rate is detected. Alternatively the signal amplitude is compared to one or more predetermined thresholds or amplitude ranges at one or more pre-determined times or during pre-determined time windows. An exponential return of the signal amplitude to a previous mean signal amplitude is detected by comparing the signal amplitude to the known step response of the first filter for the respective amplitude step. A simplified method to detect the exponential return comprises the determination of time between a the abrupt change of the signal amplitude a previous mean signal amplitude. The device may further comprise a second filter to filter the first filtered signal to a second filtered signal and a signal processing unit to detect body signals in the second filtered signal. Detected body signals are marked as invalid if a lead failure condition is indicated by the lead failure detection unit if the detected signal characteristics correspond to the step response of the first filter.

In one exemplary embodiment, the electrode is in contact with cardiac tissue. The electrode is part of a cardiac lead that is connected to an IMD like a heart monitor or a heart stimulator such as pacemakers, implantable cardioverter defibrillators (ICD) or a device for cardiac resynchronization therapy (CRT) that detects cardiac signals.

The drawings illustrate generally, by way of example, but not by way of limitation, various embodiments of the invention.
- Figure 1: is an illustration of a heart stimulation or heart monitoring system
- Figure 2: is an illustration of an example embodiment of an IMD
- Figure 3: is an illustration of atrial and ventricular electrocardiograms in one embodiment
- Figure 4: is an illustration of atrial and ventricular electrocardiograms in a further embodiment
- Figure 5: is an illustration of atrial and ventricular electrocardiograms in a further embodiment
- Figure 6: is an illustration of atrial and ventricular electrocardiograms in a further embodiment

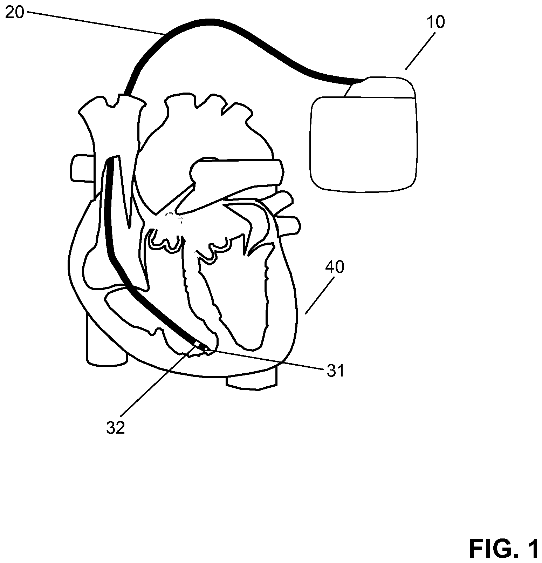

Figure 1 shows a heart stimulation or heart monitoring system comprising an IMD 10 in form of a heart stimulator or heart monitor that is connected to a cardiac lead 20. The distal end of the cardiac lead 20 comprises one or more electrodes 31 and 32 to pick up electrical signals of the heart 40.

Heart stimulators and heart monitors as shown schematically in Figure 1 are generally known in the art. The sensing of electrical activity of the heart in such devices can be performed in different ways. As shown in Figure 1 electrical cardiac signals can be sensed between two electrodes 31 and 32 at the distal end of the cardiac lead 20. This is known as bipolar sensing. Alternatively, electrical cardiac signals can be sensed between one electrode 31 or 32 at the distal end of the cardiac lead 20 and the electrically conductive housing of the IMD 10. This is known as unipolar sensing. The IMD may be connected to multiple leads each having at least one electrode that extend to different tissue locations of the heart.

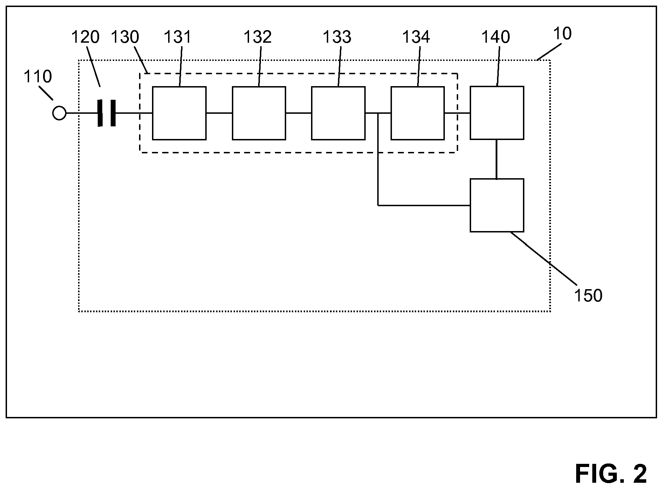

Figure 2 shows parts of the further structure of the IMD 10.
The IMD 10 comprises at least one electrode connection 110 that is coupled by a capacitor 120 to the input of a sensing unit 130 and optionally to the output of a stimulation unit (not illustrated in figure 2). The capacitor 120 and input resistors of the sensing unit form a first filter. In the preferred embodiment the first filter is a high-pass filter. Alternatively, the capacitor 120 is replaced by an analogue filter. The sensing unit 130 outputs different signals to a signal processing unit 140 and to a lead failure detection unit 150 which will described in detail hereafter. A control unit (not illustrated in figure 2) is connected to the sensing unit 130, the optional stimulation unit, the signal processing unit 140 and to the lead failure detection unit 150 to control their operation. The control unit may comprise or be connected to a memory for storage of operation parameters, operation commands and signals received from the other units of the IMD 10. The signal processing unit 140 and the lead failure detection unit 150 may also be in direct connection. The cardiac stimulator additionally includes a clock generator (not illustrated in figure 2), which provides clock signals.

Furthermore, the IMD 10 includes an electric energy source (not illustrated in figure 2), for example in the form of a battery or an accumulator, which supplies energy to the components of the IMD 10.

The electric signal received by the electrode runs through capacitor 120 to an analogue pre-amplifier 131 and an optional analogue filter 132 and is then converted by means of an A/D converter 133 into a digital signal. In an alternative embodiment (not illustrated), the electric signal received by the electrode first runs through the analogue filter and is then fed via the pre-amplifier to the A/D converter. The analogue pre-amplifier 131 and the analogue filter 132 are not absolutely necessary for the execution of the invention and may optionally also be omitted. The digital signal at the output of the A/D converter 133 is a wideband filtered time-discrete and amplitude-discrete representation of the analogue input signal of the A/D converter 133 and is then forwarded to the lead failure detection unit 150 and to a second filter 134. The output signal of the second filter 134 forms a second filtered signal that is fed to a signal processing unit 140 for detection of physiological events. For example, electric signals from an electrode placed in the ventricle that are above a specific amplitude threshold value are identified as natural contraction of the ventricle and are forwarded to the control unit. The IMD may comprise multiple sensing units, processing units and lead detection units if multiple leads are connected, for example separate units for a right atrial lead and for a right ventricular lead.

The lead failure detection unit 150 receives the wideband filtered output signal of the A/D converter 133 and analyzes the signal to detect lead failures.

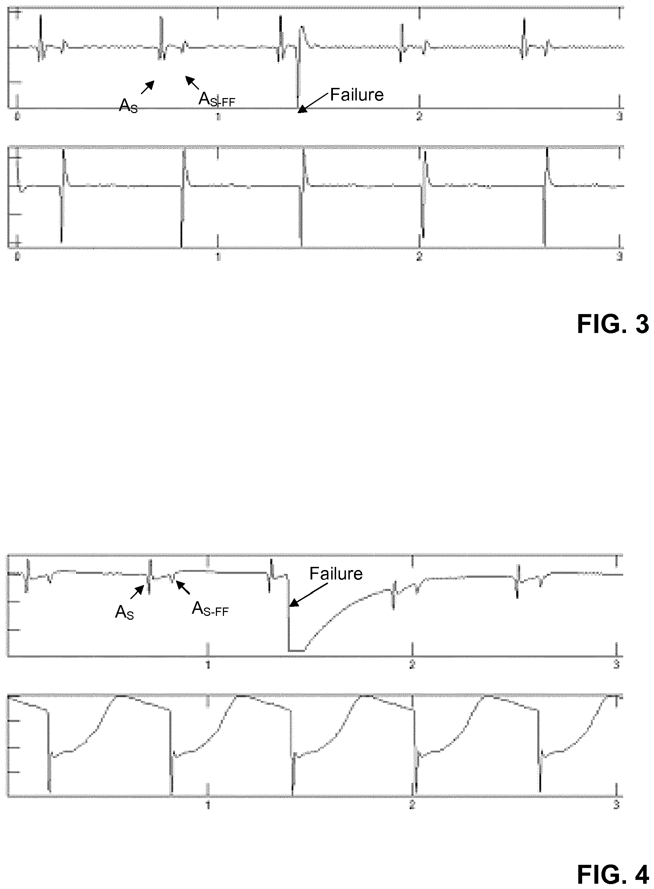

Figure 3 shows a right atrial (upper trace) and a right ventricular (lower trace) electrocardiogram of atrial and ventricular signals over time during ventricular pacing at the output of the second filter 134. Figure 4 shows the same right atrial (upper trace) and right ventricular (lower trace) electrocardiogram, but wideband filtered at the output of the A/D converter 133. In Figure 3 and Figure 4 As indicates a sensed atrial event and A_{S-FF} indicates a far-field sensing of the ventricular depolarization. Failure indicates the point of time where the atrial electrode was in contact to the housing for a short time. In the filtered signal shown in Figure 3 the short appears as a peak similar to a sensed atrial event, having an amplitude significantly higher than the far field signal and may therefore be misinterpreted as sensed atrial event. Figure 4 shows, that in the wideband filtered signal the short is represented by an abrupt drop of the atrial signal amplitude followed by an exponential return to the previous mean signal amplitude.

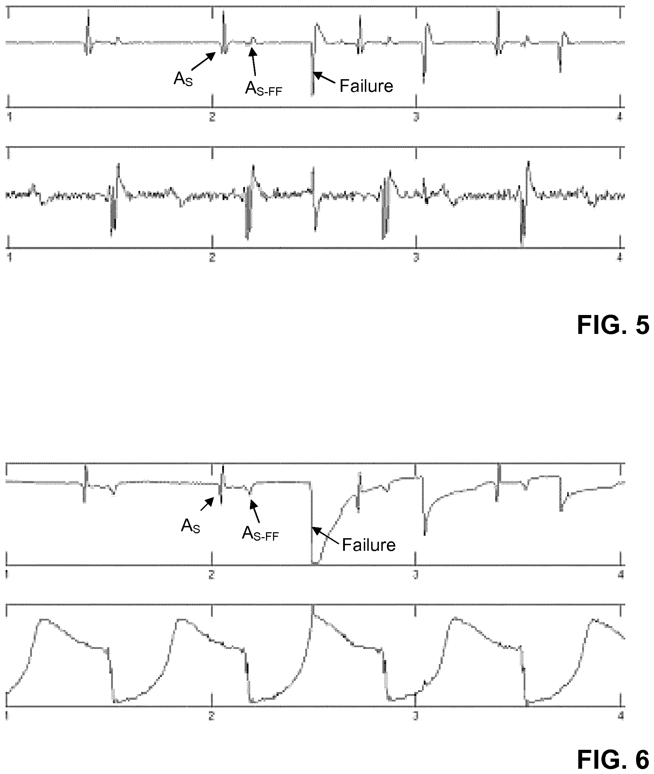

Figure 5 shows a right atrial (upper trace) and a right ventricular (lower trace) electrocardiogram of atrial and ventricular signals over time without pacing at the output of the second filter 134. Figure 6 shows the same right atrial (upper trace) and right ventricular (lower trace) electrocardiogram, but wideband filtered at the output of the A/D converter 133. In Figure 5 and Figure 6 As indicates a sensed atrial event and A_{S-FF} indicates a far-field sensing of the ventricular intrinsic event. Failure indicates the point of time where the atrial electrode was in contact to another electrode for a short time. In the filtered signal shown in Figure 5 the short again appears as a peak similar to a sensed atrial event, having a amplitude significantly higher than the far field signal and may therefore be misinterpreted as sensed atrial event. Figure 6 shows, that in the wideband filtered signal the short is represented by a abrupt drop of the atrial signal amplitude followed by a exponential return to the previous signal amplitude.

As shown in Figures 3, 4, 5 and 6, lead failures cause significant signal morphologies in the wideband filtered analog-digital converted signal. Lead failures like shorts or interruptions cause impulses at the input connector 110. The first filter formed by the capacitor 120 and input resistors forms a high-pass filter. The step response can be detected at the output of the A/D converter 133. The characteristic signal features of a lead failure condition are an abrupt change of the signal amplitude followed by an exponential return to the previous mean signal amplitude.

Lead failure detection unit 150 is configured to continuously analyze the output signal of the A/D converter 133 for the occurrence of such characteristic signal features. In one embodiment, an abrupt change of the signal amplitude is detected by analyzing the slew rate of the signal amplitude. In a preferred embodiment, a minimum slew rate is detected. Alternatively the signal amplitude is compared to a one or more predetermined thresholds or amplitude ranges at one or more pre-determined times or during pre-determined time windows. The thresholds may be adaptive, for example a percentage of the mean or maximal or averaged signal amplitude. The times or time-windows may also be adaptive, for example depending of the heart rate.

The return to the previous signal level is slower than any intrinsic cardiac signal and is detected by by comparing the signal amplitude to the known step response of the first filter for the respective amplitude step. A simplified method to detect the exponential return comprises the determination of time between the abrupt change of the signal amplitude and the return to a previous mean signal amplitude.

If signal features characteristic for a lead failure condition are detected, lead failure detection unit 150 indicates a lead failure condition.

Signal processing unit (140) is configured to continuously analyze the output signal of filter 134 to detect body signals in the second filtered signal. In one embodiment signal processing unit (140) is configured to detect cardiac signals or cardiac events like P-waves, QRS-complexes or T-waves using techniques known in the art. If a body signal is detected and lead failure detection unit 150 indicates at the same time a failure condition, the detected body signal - in this embodiment the detected cardiac signals - are marked invalid. By this, a wrong diagnosis or wrong therapy decision can be avoided.

## Claims

1. A method to detect a lead failure condition for a lead having at least one electrode in contact with body tissue comprising the steps of:
- sensing a electric signal from the body tissue using said electrode,
- filtering the sensed electric signal using a first filter to a first filtered signal,
- detecting signal characteristics of the first filtered signal;
**characterized by** the step of
- indicating a lead failure condition if said detected signal characteristics correspond to the step response of said first filter.

2. The method of claim 1, **characterized in that** the first filter is a high-pass filter.

3. The method of claim 2, **characterized in that** the step of detecting signal characteristics comprises the detection of an abrupt change of the signal amplitude and the detection of an exponential return of the signal amplitude to a previous mean signal amplitude.

4. The method of claim 3, **characterized in that** the step of detection of an abrupt change of the signal amplitude comprises the step of detecting a minimum slew rate of the signal amplitude.

5. The method of claim 3, **characterized in that** an exponential return of the signal amplitude to a previous mean signal amplitude is detected if the time between the abrupt change of the signal amplitude and said return exceeds a predetermined time.

6. The method of claim 1, **characterized by** the additional steps of:
- filtering the first filtered signal using a second filter to a second filtered signal
- detecting body signals in the second filtered signal
- marking detected body signals as invalid if a lead failure condition is indicated.

7. The method of claim 1, **characterized in that** the electric signals are cardiac signals.

8. A device (10), configured to detect a lead failure condition for a lead (20) having at least one electrode (31, 32) in contact with body tissue wherein the lead (20) is connected to the device (10) comprising:
- a first filter (120) to filter a electric signal sensed by the at least one electrode (31, 32) to a first filtered signal
- a lead failure detection unit (150) that is configured to detect signal characteristics of the first filtered signal
**characterized in that**
a lead failure condition is indicated by the lead failure detection unit (150) if said detected signal characteristics correspond to the step response of said first filter (120).

9. The device (10) of claim 8, **characterized in that** the first filter (120) is a high-pass filter.

10. The device (10) of claim 9, **characterized in that** the signal characteristics comprise an abrupt change of the signal amplitude and an exponential return of the signal amplitude to a previous mean signal amplitude.

11. The device (10) of claim 10, **characterized in that** an abrupt change of the signal amplitude is detected by detecting a minimum slew rate of the signal amplitude.

12. The device (10) according to claim 10, **characterized in that** an exponential return of the signal amplitude to a previous mean signal amplitude is detected if the time between the abrupt change of the signal amplitude and said return exceeds a predetermined time.

13. The device (10) according to claim 8, further comprising:
- a second filter (134) to filter the first filtered signal to a second filtered signal,
- a signal processing unit (140) to detect body signals in the second filtered signal; **characterized in that** detected body signals are marked as invalid if a lead failure condition is indicated.

14. The device (10) according to claim 8, **characterized in that** the electric signals are cardiac signals.

## Patentansprüche

1. Verfahren zum Erkennen eines Leitungsdefektzustands für eine Leitung mit mindestens einer Elektrode in Kontakt mit Körpergewebe, das folgende Schritte umfasst:
- Erfassen eines elektrischen Signals vom Körpergewebe unter Verwendung der Elektrode,
- Filtern des erfassten elektrischen Signals unter Verwendung eines ersten Filters zu einem ersten gefilterten Signal,
- Feststellen von Signaleigenschaften des ersten gefilterten Signals; **gekennzeichnet durch** folgenden Schritt:
- Angeben eines Leitungsdefektzustands, wenn die festgestellten Signaleigenschaften der Sprangantwort des ersten Filters entsprechen.

2. Verfahren nach Anspruch 1, **dadurch gekennzeichnet, dass** das erste Filter ein Hochpassfilter ist.

3. Verfahren nach Anspruch 2, **dadurch gekennzeichnet, dass** der Schritt des Feststellens von Signaleigenschaften das Erkennen einer abrupten Änderung der Signalamplitude und das Erkennen einer exponentiell verlaufenden Rückkehr der Signalamplitude zu einer vorherigen mittleren Signalamplitude umfasst.

4. Verfahren nach Anspruch 3, **dadurch gekennzeichnet, dass** der Schritt des Erkennens einer abrupten Änderung der Signalamplitude den Schritt des Erkennens einer minimalen Flankensteilheit der Signalamplitude umfasst.

5. Verfahren nach Anspruch 3, **dadurch gekennzeichnet, dass** eine exponentiell verlaufende Rückkehr der Signalamplitude zu einer vorherigen mittleren Signalamplitude erkannt wird, wenn die Zeit zwischen der abrupten Änderung der Signalamplitude und der Rückkehr länger ist als eine vorher festgelegte Zeit.

6. Verfahren nach Anspruch 1, **gekennzeichnet durch** folgende zusätzliche Schritte:
- Filtern des ersten gefilterten Signals unter Verwendung eines zweiten Filters zu einem zweiten gefilterten Signal,
- Erkennen von Körpersignalen in dem zweiten gefilterten Signal,
- Kennzeichnen erkannter Körpersignale als ungültig, wenn ein Leitungsdefektzustand angegeben wird.

7. Verfahren nach Anspruch 1, **dadurch gekennzeichnet, dass** die elektrischen Signale Herzsignale sind.

8. Vorrichtung (10), die so ausgelegt ist, dass sie einen Leitungsdefektzustand für eine Leitung (20) mit mindestens einer Elektrode (31, 32) in Kontakt mit Körpergewebe erkennt, wobei die Leitung (20) mit der Vorrichtung (10) verbunden ist, die Folgendes umfasst:
- ein erstes Filter (120) zum Filtern eines elektrischen Signals, das von der mindestens einen Elektrode (31, 32) erfasst wird, zu einem ersten gefilterten Signal,
- eine Leitungsdefekterkennungseinheit (150), die so ausgelegt ist, dass sie Signaleigenschaften des ersten gefilterten Signals feststellt,
**dadurch gekennzeichnet, dass**
ein Leitungsdefektzustand von der Leitungsdefekterkennungseinheit (150) angegeben wird, wenn die festgestellten Signaleigenschaften der Sprungantwort des ersten Filters (120) entsprechen.

9. Vorrichtung (10) nach Anspruch 8, **dadurch gekennzeichnet, dass** das erste Filter (120) ein Hochpassfilter ist.

10. Vorrichtung (10) nach Anspruch 9, **dadurch gekennzeichnet, dass** die Signaleigenschaften eine abrupte Änderung der Signalamplitude und eine exponentiell verlaufende Rückkehr der Signalamplitude zu einer vorherigen mittleren Signalamplitude umfassen.

11. Vorrichtung (10) nach Anspruch 10, **dadurch gekennzeichnet, dass** eine abrupte Änderung der Signalamplitude durch Erkennen einer minimalen Flankensteilheit der Signalamplitude erkannt wird.

12. Vorrichtung (10) nach Anspruch 10, **dadurch gekennzeichnet, dass** eine exponentiell verlaufende Rückkehr der Signalamplitude zu einer vorherigen mittleren Signalamplitude erkannt wird, wenn die Zeit zwischen der abrupten Änderung der Signalamplitude und der Rückkehr länger ist als eine vorher festgelegte Zeit.

13. Vorrichtung (10) nach Anspruch 8, ferner umfassend:
- ein zweites Filter (134) zum Filtern des ersten gefilterten Signals zu einem zweiten gefilterten Signal,
- eine Signalverarbeitungseinheit (140) zum Erkennen von Körpersignalen in dem zweiten gefilterten Signal;
**dadurch gekennzeichnet, dass** erkannte Körpersignale als ungültig gekennzeichnet werden, wenn ein Leitungsdefektzustand angegeben wird.

14. Vorrichtung (10) nach Anspruch 8, **dadurch gekennzeichnet, dass** die elektrischen Signale Herzsignale sind.

## Revendications

1. Procédé pour détecter un état de panne de connexion pour une connexion ayant au moins une électrode en contact avec du tissu corporel, comprenant les étapes de :
- capture d'un signal électrique à partir du tissu corporel en utilisant ladite électrode,
- filtrage du signal électrique détecté en utilisant un premier filtre pour un premier signal filtré,
- détection des caractéristiques de signal du premier signal filtré ;
**caractérisé par** l'étape
- d'indication d'un état de panne de connexion si lesdites caractéristiques de signal détectées correspondent à la réponse transitoire dudit premier filtre.

2. Procédé selon la revendication 1, **caractérisé en ce que** le premier filtre est un filtre passe-haut.

3. Procédé selon la revendication 2, **caractérisé en ce que** l'étape de détection des caractéristiques de signal comprend la détection d'une variation abrupte de l'amplitude du signal et la détection d'un retour exponentiel de l'amplitude de signal vers une amplitude de signal moyenne précédente.

4. Procédé selon la revendication 3, **caractérisé en ce que** l'étape de détection d'une variation abrupte de l'amplitude du signal comprend l'étape de détection d'une vitesse de balayage minimale de l'amplitude du signal.

5. Procédé selon la revendication 3, **caractérisé en ce qu'**un retour exponentiel de l'amplitude du signal à une amplitude de signal moyenne précédente est détectée si le temps entre la variation abrupte de l'amplitude du signal et ledit retour dépasse un temps prédéterminé.

6. Procédé selon la revendication 1, **caractérisé par** les étapes supplémentaires de :
- filtrage du premier signal filtré en utilisant un deuxième filtre pour un deuxième signal filtré
- détection de signaux corporels dans le deuxième signal filtré,
- marquage des signaux corporels détectés comme invalides si un état de panne de connexion est indiqué.

7. Procédé selon la revendication 1, **caractérisé en ce que** les signaux électriques sont des signaux cardiaques.

8. Dispositif (10), configuré pour détecter un état de panne de connexion pour une connexion (20) ayant au moins une électrode (31, 32) en contact avec du tissu corporel, où la connexion (20) est connectée au dispositif (10) comprenant :
- un premier filtre (120) pour filtrer un premier signal capté par l'au moins une électrode (31, 32) pour un premier signal filtré
- une unité de détection de panne de connexion (150) qui est configurée pour détecter des caractéristiques de signal du premier signal filtré
**caractérisé en ce**
**qu'**un état de panne de connexion est indiqué par l'unité de détection de la panne de connexion (150) si lesdites caractéristiques de signal détectées correspondent à la réponse transitoire dudit premier filtre (120).

9. Dispositif (10) selon la revendication 8, **caractérisé en ce que** le premier filtre (120) est un filtre passe-haut.

10. Dispositif (10) selon la revendication 9, **caractérisé en ce que** les caractéristiques de signal comprennent une variation abrupte de l'amplitude de signal et un retour exponentiel de l'amplitude de signal vers une amplitude de signal moyenne précédente.

11. Dispositif (10) selon la revendication 10, **caractérisé en ce qu'**une variation abrupte de l'amplitude de signal est détectée en détectant une vitesse de balayage minimale de l'amplitude de signal.

12. Dispositif (10) selon la revendication 10, **caractérisé en ce qu'**un retour exponentiel de l'amplitude de signal vers une amplitude de signal moyenne précédente est détecté si le temps entre la variation abrupte de l'amplitude de signal et ledit retour dépasse un temps prédéterminé.

13. Dispositif (10) selon la revendication 8, comprenant en outre :
- un deuxième filtre (134) pour filtrer le premier signal filtré pour un deuxième signal filtré,
- une unité de traitement de signal (140) pour détecter des signaux corporels dans le deuxième signal filtré ;
**caractérisé en ce que** des signaux corporels détectés sont marqués invalides si un état de panne de connexion est indiqué.

14. Dispositif (10) selon la revendication 8, **caractérisé en ce que** les signaux électriques sont des signaux cardiaques.
